# EUROPEAN PATENT APPLICATION

(11) **EP 3 988 013 A1**
(43) Date of publication of application: **27.04.2022**
(21) Application number: 21202944.1
(22) Date of filing: 15.10.2021
(51) Int. Cl.: A61B 5/00, A61B 5/024, A61B 5/026, A61B 5/1455

(54) **WEARABLE OPTOELECTRONIC SENSING DEVICE AND MANUFACTURING METHOD THEREOF**

(30) Priority: 16.10.2020 US 202063092687 P
(71) Applicant: Amengine Corporation, Hsinchu 302 (TW)
(72) Inventor: Hsu, Chia Liang, 302 Hsinchu (TW); Shen, Jian Yu, 302 Hsinchu (TW)
(74) Representative: Ter Meer Steinmeister & Partner

(57) **Abstract**

A wearable optoelectronic sensing device includes a textile layer, a substrate layer, a light emitter, a plurality of light receivers, and a cover layer. The substrate layer is disposed on the textile layer. The plurality of light receivers is disposed on the surface of the substrate layer and formed as an array. The light emitter is disposed at a geometric center of the array. The plurality of light receiver includes a first light receiver and a second light receiver. A sensing wavelength of the second light receiver is greater than that of the first light receiver. A distance between the second light receiver and the light emitter is greater than a distance between the first light receiver and the light emitter. The cover layer is disposed on the substrate layer and includes a plurality of openings. The position of each opening corresponds to position of the light emitter and the position of each light receiver.

## Description

### BACKGROUND

### Technical Field

The instant disclosure relates to a wearable device and a manufacturing method thereof, in particular, to a wearable device with an optoelectronic sensor and a manufacturing method thereof.

### Related Art

At present, common health detection devices in the market include watches, bracelets, earphones, mobile phones, portable detection devices, and other electronic products. Although a variety of physiological data measurement technologies have been applied to various electronic products, the accuracy of these measurement technologies is still insufficient, resulting in the inability to accurately determine the real physiological conditions. As a result, some consumers would not pay for these products, and these products also fail to provide reliable measurement for doctors as diagnostic reference. Therefore, considering the future development trend of medical devices, how to effectively improve the signal-to-noise ratio of detection devices is a problem to be solved.

### SUMMARY

In view of this, a wearable optoelectronic sensing device and a manufacturing method thereof are provided. In one embodiment, the wearable optoelectronic sensing device includes a textile layer, a substrate layer, a light emitter, a plurality of light receivers, and a cover layer. The substrate layer is disposed on the textile layer. The light emitter is disposed on a surface of the substrate layer. The plurality of light receiver is disposed on the surface of the substrate layer and formed in an array, and the light emitter is disposed at a geometric center of the array. The plurality of light receiver includes a first light receiver and a second light receiver. The first light receiver is adapted to sense a first sensing wavelength. A first distance is between the first light receiver and the light emitter. The second light receiver is adapted to sense a second sensing wavelength greater than the first sensing wavelength. A second distance is between the second light receiver and the light emitter, and the second distance is greater than the first distance. The cover layer is disposed on the substrate layer, so that the substrate layer is between the textile layer and the cover layer. The cover layer includes a plurality of openings. The position of each opening corresponds to the position of the light emitter and the position of each light receiver.

The wearable optoelectronic sensing device could be further combined with one or more of the following optional features.

In one or more embodiments, the wearable optoelectronic sensing device may further comprise a transparent material layer disposed on the cover layer.

In one or more embodiments a refractive index of the transparent material layer may be in a range between 1.42 and 1.55.

In one or more embodiments, the wearable optoelectronic sensing device may further comprise a plurality of transparent protruding dots disposed on the cover layer

In one or more embodiments, each of the plurality of transparent protruding dots may have a position corresponding to at least one of the openings.

In one or more embodiments, the wearable optoelectronic sensing device may further comprise a physical therapeutic device.

In one or more embodiments, the wearable optoelectronic sensing device may further comprise a communication module.

In one or more embodiments, the light emitter may be coupled to the communication module.

In one or more embodiments, the physical therapeutic device may comprise a therapeutic light emitter and a therapeutic light receiver.

In one or more embodiments, the light emitter may be adapted to emit a first light.-

In one or more embodiments, the therapeutic light emitter may be adapted to emit a second light which has an emission wavelength different from that of the first light.

In one or more embodiments, the emission wavelength of the second light may be in a range between 600 nm and 640 nm.

In one or more embodiments, the physical therapeutic device may comprise a plurality of therapeutic light emitters disposed on the surface of the substrate layer.

In one or more embodiments, the plurality of therapeutic light emitters may be configured to emit light to an acupoint of the user.

In one or more embodiments, the wearable optoelectronic sensing device may be disposed on a knee cap.

In one or more embodiments, the physical therapeutic device may be a heater.

In one or more embodiments, the light emitter and the plurality of light receivers may be disposed on a front side of the knee cap.

In one or more embodiments, the heater may be disposed on a rear side of the knee cap.

In one or more embodiments, the heater may be one of an elongated, spiral, or curved structure extending along a longitudinal axis of the knee cap.

In one or more embodiments, the textile layer may be formed as a closed loop structure which comprises a receiving space and two connecting openings.

In one or more embodiments, the two connecting openings may be opposite to each other.

In one or more embodiments, each connecting opening may comprise two sets of connection members, and the substrate layer is disposed in the receiving space.

In one or more embodiments, the wearable optoelectronic sensing device may be disposed on a cuff.

In one or more embodiments, the wearable optoelectronic sensing device may further comprise a controller and/or a pressure sensor.

In one or more embodiments, the controller may be adapted to receive a light sensing signal from at least one of the plurality of light receivers.

In one or more embodiments, the controller may be adapted convert the light sensing signal into a blood pressure signal according to a conversion equation.

In one or more embodiments, the controller may be adapted receive a pressure sensing signal from the pressure sensor.

In one or more embodiments, the controller may be adapted calibrate the conversion equation according to the pressure sensing signal.

In one or more embodiments, the controller may be adapted to adjust a plurality of pressure states of the cuff.

In one or more embodiments, the controller may be adapted to receive the light sensing signal from at least one of the light receivers and receive the pressure sensing signal from the pressure sensor in each of the pressure states.

In one or more embodiments, the controller may be adapted to calibrate the conversion equation according to the pressure sensing signal.

In one or more embodiments, the wearable optoelectronic sensing device may further comprise a flexible panel disposed on the surface of the substrate layer.

In one or more embodiments, the first sensing wavelength may be 525 nm and/or the second sensing wavelength may be 660 nm.

In one or more embodiments, the plurality of light receivers may comprise a third light receiver adapted to sense a third sensing wavelength which is 940 nm.

In one or more embodiments, a third distance greater than the second distance may be between the third light receiver and the light emitter.

### BRIEF DESCRIPTION OF THE DRAWINGS

The disclosure will become more fully understood from the detailed description given herein below for illustration only, and thus not limitative of the disclosure, wherein:
FIG. 1 is a cross-sectional view of a wearable optoelectronic sensing device according to an embodiment of the instant disclosure;
FIGS. 2A to 2F are top views of wearable optoelectronic sensing devices according to different embodiments of the instant disclosure;
FIGS. 3A to 3F is top views of wearable optoelectronic sensing devices according to another exemplary embodiment of the instant disclosure;
FIG. 4A and FIG. 4B are PPG graphs of wearable optoelectronic sensing devices according to different embodiments of the instant disclosure;
FIG. 5 is a block diagram of a wearable optoelectronic sensing device according to an exemplary embodiment of the instant disclosure;
FIG. 6 is a operational view of a wearable optoelectronic sensing device according to an exemplary embodiment of the instant disclosure;
FIGS. 7A to 7C are views of a knee cap having a wearable optoelectronic sensing device according to an exemplary embodiment of the instant disclosure;
FIGS. 7D to 7F are rear views of knee caps having wearable optoelectronic sensing devices according to different embodiments of the instant disclosure;
FIG. 8 is an operational flowchart of a wearable optoelectronic sensing device according to an exemplary embodiment of the instant disclosure;
FIG. 9A and FIG. 9B are views of a connection member of the knee cap according to an exemplary embodiment of the instant disclosure;
FIG. 10 is a operational view of a kerchief having a wearable optoelectronic sensing device according to an exemplary embodiment of the instant disclosure;
FIG. 11A is a view of a cuff having a wearable optoelectronic sensing device according to an exemplary embodiment of the instant disclosure;
FIG. 11B is a block diagram of a wearable optoelectronic sensing device according to another exemplary embodiment of the instant disclosure;
FIG. 11C is a circuit block diagram of a cuff having a wearable optoelectronic sensing device according to an exemplary embodiment of the instant disclosure;
FIG. 11D is a flowchart of a calibration procedure of the cuff having the wearable optoelectronic sensing device according to an exemplary embodiment of the instant disclosure;
FIG. 11E is a flowchart of a measurement quality adjustment procedure of the cuff having the wearable optoelectronic sensing device according to an exemplary embodiment of the instant disclosure;
FIG. 11F is a graph showing signals before and after the measurement quality adjustment procedure according to an exemplary embodiment of the instant disclosure;
FIG. 12 is a view of a wearable optoelectronic sensing device having a display panel according to an exemplary embodiment of the instant disclosure; and
FIG. 13 is a manufacturing flowchart of a wearable optoelectronic sensing device according to an exemplary embodiment of the instant disclosure.

### DETAILED DESCRIPTION

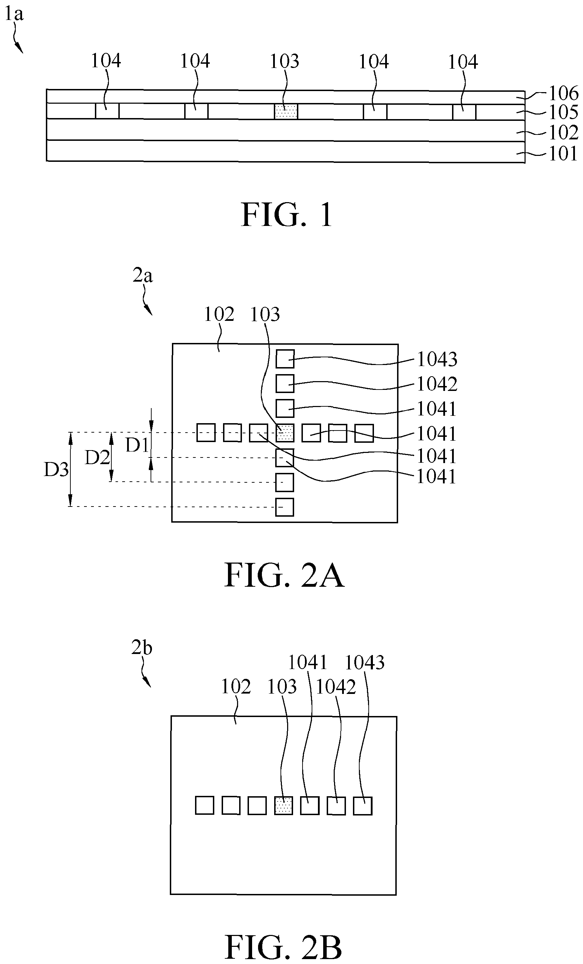

FIG. 1 is a cross-sectional view of a wearable optoelectronic sensing device according to an exemplary embodiment of the instant disclosure. In this embodiment, the wearable optoelectronic sensing device 1a is adapted to be attached to the surface of skin of an organism (e.g., the human body) for biometric recognition, or is adapted to sense a physiological signal of the organism by photoplethysmography (PPG). The physiological signal may be blood oxygen concentration, muscle oxygen concentration, brain oxygen concentration, heartrate, blood pressure, lactic acid concentration, atrial fibrillation, water in the human body, blood sugar concentration, body temperature, and blood flow rate. In this embodiment, the wearable optoelectronic sensing device 1a includes a textile layer 101, a substrate layer 102, a light emitter 103, a plurality of light receivers 104, a cover layer 105, and a transparent material layer 106. The substrate layer 102 is disposed on the textile layer 101. In one embodiment, the substrate layer 102 is a bendable flexible substrate. The cover layer 105 is disposed on the substrate layer 102, so that the substrate layer 102 is between the textile layer 101 and the cover layer 105. The light emitter 103 and the plurality of light receivers 104 are disposed on the substrate layer 102 and electrically connected to the substrate layer 102. The cover layer 105 includes a plurality of openings, and position of each opening corresponds to the position of the light emitter 103 and the position of each light receiver 104. The light emitter 103 and the plurality of light receivers 104 are arranged in the openings, and the cover layer 105 surrounds the light emitter 103 and the plurality of light receivers 104. In one embodiment, the topmost surface of the light emitter 103, the topmost surfaces of the plurality of light receivers 104, and the topmost surface of the cover layer 105 are coplanar. In another embodiment, the topmost surface of the light emitter 103 and the topmost surfaces of the plurality of light receivers 104 are not flushed with the topmost surface of the cover layer 105. For example, the topmost surface of the light emitter 103 and the topmost surfaces of the plurality of light receivers 104 may be higher than or lower than the topmost surface of the cover layer 105. The transparent material layer 106 covers the light emitter 103, the plurality of light receivers 104, and the cover layer 105. The transparent material layer 106 is adapted to protect the light emitter 103 and the plurality of light receivers 104. Optionally, the wearable optoelectronic sensing device 1a may further include an electronic component (not shown)which can be an integrated circuit (IC), an electrocardiography (ECG) sensor, a power module, a thermotherapeutic module, a phototherapeutic module, a piezoelectric sensor, an ultraviolet (UV) sterilization device, a gravity sensor (G sensor), a display module, etc.

The textile layer 101 may be, but not limited to, nonwoven fabric, cotton, linen, wool, silk, rayon, nylon, polyester, acrylic fiber, cellulose acetate (CA) fiber, triacetate fiber, elastic fiber, fiberglass, metal fiber, rubber fiber, or fabric made of blended fibers. The substrate layer 102 may be a flexible circuit board. The material of the substrate layer 102 may be polyimide, polyester film (PET), bismaleimide triazine (BT), or Ajinomoto build-up film (ABF). The substrate layer 102 comprises wires electrically connected to the light emitter 103, the plurality of light receivers 104, or other electronic components. In one embodiment, in the case that the electronic component is a thermotherapeutic module, the substrate layer 102 may further include a heatable metal wire for heating the entire wearable optoelectronic sensing device to provide the thermotherapeutic treatment. The material of the heatable metal wire may be a metal wire with high impendence, for example, tungsten, nickel chromium alloy, iron chromium alloy, copper nickel alloy, or copper manganese alloy. The cover layer 105 is heat resistant, water resistant, and has some permeability, and the cover layer 105 is adapted to position the light emitter 103 and the light receivers 104 on the substrate layer 102. The cover layer 105 may include at least one selected from the group consisting of germanium fabrics, polyimide (PI), polyester film, silastic, mica sheet, thermoplastic polyurethane (TPU), polytetrafluoroethene (PTFE), benzocyclobutene (BCB), perfluorocyclobutane (PFCB), epoxy resin, Su8 photoresist, and spin-on glass (SOG). The transparent material layer 106 is adapted to protect the light emitter 103 and the plurality of light receivers 104 from contacting the skin of the organism. The material of the transparent material layer 106 may include at least one selected from the group consisting of thermoplastic polyurethane (TPU), acrylic, poly(methyl methacrylate) (PMMA), thermoplastic rubber, silicone, epoxy resin, polyimide (PI), benzocyclobutene (BCB), perfluorocyclobutane (PFCB), Su8 photoresist, polyethylene terephthalate (PET), polycarbonate (PC), polyetherimide, fluorocarbon polymer, aluminum oxide (Al₂O₃), siloxane polymer (SINR), and spin-on glass (SOG). In one embodiment, a refractive index of the transparent material layer 106 is in a range between 1.42 and 1.55. The light emitter 103, the plurality of light receivers 104, or the other electronic components may be adhered on the substrate layer 102 through adhesive material, or may be positioned on the substrate layer 102 through flip bonding or wire bonding. The adhesive material may include at least one selected from the group consisting of silver paste, polyimide, benzocyclobutene (BCB), perfluorocyclobutane (PFCB), epoxy resin, Su8 photoresist, and spin-on glass (SOG). The light receiver 104 may be, but not limited to, a photodiode, a photoresistor, or a visible or invisible light sensor. The light emitter 103 may be, but not limited to, a laser diode (LD), an organic light emitting diode (OLED), or an LED (micro LED). The LED may be a chip with a single diode, a chip with an array of diode units (an LED that can be operated under high voltage), or a micro LED array. The photodiode may be a chip with a single diode or a chip with an array of diode units.

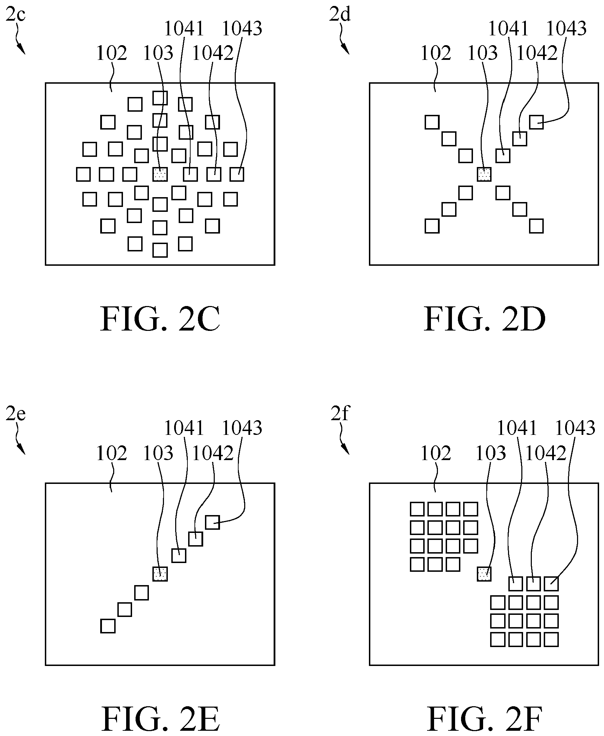

FIG. 2A to FIG. 2F illustrate top views of wearable optoelectronic sensing devices according to different embodiments of the instant disclosure. In one embodiment, the wearable optoelectronic sensing device 2a-2f has a light emitter 103 and a plurality of light receivers 104 disposed on the surface of the substrate layer 102.The plurality of light receivers 104 is symmetrically arranged around the light emitter 103 as an array to increase the sensitivity of the plurality of light receivers 104. In this embodiment, the array is arranged in a regular arrangement pattern and is not limited to the square matrix. As shown in FIG. 2A, the plurality of light receivers 104 of the wearable optoelectronic sensing device 2a is arranged as an array which has a "+" profile. Specifically, in this embodiment, the plurality of light receivers 104 is formed in two linear arrays perpendicular to each other, and the light emitter 103 is disposed at the intersection of the two linear arrays. As shown in FIG. 2B, the plurality of light receivers 104 of the wearable optoelectronic sensing device 2b is arranged as an array, and the array has a "—" profile. In other words, in this embodiment, the plurality of light receivers 104 is formed in a single linear array, and the light emitter 103 is disposed on a center portion of the linear array. As shown in FIG. 2C, the plurality of light receivers 104 of the wearable optoelectronic sensing device 2c is arranged as an array, the array has a profile of concentric circles, and the light emitter 103 is disposed at the center of circle of the concentric circles. As shown in FIG. 2D, the plurality of light receivers 104 of the wearable optoelectronic sensing device 2d is arranged as an array, and the array has an "X" profile. In other words, in this embodiment, the plurality of light receivers 104 is formed in two linear arrays perpendicular to each other and extending diagonally, the angle between the two linear arrays may not be equal to 90 degrees, and the light emitter 103 is disposed at the intersection of the two linear arrays. As shown in FIG. 2E, the plurality of light receivers 104 of the wearable optoelectronic sensing device 2e is arranged as an array, and the array has a tilted "—" profile. In other words, in this embodiment, the plurality of light receivers is arranged as a single linear array, the linear array is not parallel to any of sides of the substrate layer, and the light emitter 103 is disposed at a center portion of the linear array. As shown in FIG. 2F, the plurality of light receivers 104 of the wearable optoelectronic sensing device 2f is arranged as two rectangular arrays, and the two rectangular arrays are of a point symmetry with respect to the light emitter 103. In general, in these embodiments, the light emitter 103 is disposed at a geometric center of two arrays formed by the plurality of light receivers 104. Taking the wearable optoelectronic sensing device 2a shown in FIG. 2A as an example, four first light receivers 1041 are arranged at four sides of the light emitter 103 respectively. A first distance D1 is between each of the first light receivers 1041 and the light emitter 103. When the light beam emitted by the light emitter 103 is not blocked, the light intensities received by the four first light receivers 1041 are the same. Similarly, in the device shown in FIG. 2A to FIG. 2F, the plurality of light receivers 104 disposed at four sides of the light emitter 103 and equidistantly spaced from the light emitter 103 receives lights with the same light intensity.

In one embodiment, the light emitter 103 of the wearable optoelectronic sensing device is adapted to emit light having multiple emission wavelength, and the plurality of light receivers is adapted to receive different lights with different wavelengths. Hence, the wearable optoelectronic sensing device can detect different physiological signals by sensing different lights (lights having different wavelengths). Taking the device shown in FIG. 2A as an example, the wearable optoelectronic sensing device 2a includes a light emitter 103, a plurality of first light receivers 1041, a plurality of second light receivers 1042, and a plurality of third light receivers 1043. The light emitter 103 is adapted to emit lights having at least three emission wavelengths. Lights with longer wavelength can penetrate the skin with a deeper depth, and be reflected along the light path which is comparatively distant from the light emitter 103. Lights with shorter wavelength can penetrate the skin with a shallower depth, and be reflected along the light path which is comparatively close to the light emitter 103. Therefore, the plurality of light receivers 104 adapted to sense lights having a shorter sensing wavelength is disposed at positions nearer to the light emitter 103, and the plurality of light receivers 104 adapted to sense lights having a longer sensing wavelength is disposed at positions farer from the light emitter 103. Based on this arrangement, different light with different wavelength can be received by the light receivers. Hence, the sensing capability of the wearable optoelectronic sensing device 2a which is applied to monitor different physiological signals is improved Specifically, in this embodiment, a first distance D1 is between the first light receiver 1041 and the light emitter 103, a second distance D2 is between the second light receiver 1042 and the light emitter 103, and a third distance D3 is between the third light receiver 1043 and the light emitter 103. The third distance D3 is greater than the second distance D2, and the second distance D2 is greater than the first distance D1. The sensing wavelength of the third light receiver 1043 is greater than the sensing wavelength of the second light receiver 1042, and the sensing wavelength of the second light receiver 1042 is greater than the sensing wavelength of the first light receiver 1041. In one embodiment, the sensing wavelength can be defined as the wavelength in which the light receiver 104 has a highest response rate. In another embodiment, the sensing wavelength can be defined as the wavelength range in which the response rate of the light receiver 104 is higher than a value, which is the peak power value of the response rate distribution minus 3 dB. In one embodiment, the light emitter 103 is adapted to emit lights having at least three different wavelengths, for example, the dominant wavelength or the peak wavelength of the lights may be 525 nm, 660 nm, 940 nm, and/or 1300 nm. Each of the light receivers 1041, 1042, 1043 is adapted to sense lights having at least one sensing wavelength. Moreover, the light receivers nearer to the light emitter 103 have a shorter sensing wavelength, and the light receivers farer from the light emitter 103 have a longer sensing wavelength. For example, the sensing wavelength of the light receivers 1041 is 525 nm and/or 660 nm, the sensing wavelength of the light receivers 1042 is 660 nm and/or 940 nm, and the sensing wavelength of the light receivers 1043 is 1300 nm. In one embodiment, light having a wavelength of 525 nm is adapted to measure heartrate signals by the PPG detection or to recognize personal features in the capillaries from the epidermis to dermis in biometric recognition. Lights having a wavelength of 660 nm or 940 nm are adapted to measure blood oxygen concentration or blood pressure signal by the PPG detection or to recognize personal features in the vessels from the dermis to the subcutaneous tissues in biometric recognition. Lights having a wavelength of 1300 nm are adapted to measure the moisture content of the subcutaneous tissues by the PPG detection or to recognize personal features of the moisture content of the subcutaneous tissues in the biometric recognition.

In one embodiment, the wearable optoelectronic sensing device 1a may sequentially control the light emitter 103 to turn on or turn off the emission of the lights with different emission wavelengths, so that the wearable optoelectronic sensing device 1a sequentially monitors different physiological signals. For example, the light emitter 103 sequentially emits lights having wavelengths of 525 nm, 660 nm, and 940 nm. After the lights are sensed and received by different light receivers 104, the vessels or skin tissues with different depths are then sequentially detected for personal biometric recognition, or different types of signals in the PPG detection can be sequentially detected. Then, the heartrate, the blood oxygen concentration, the blood pressure, and/or the moisture content of the subcutaneous tissues can be measured. In another embodiment, the light emitter 103 may emit lights with different wavelengths at the same time, different physiological signals as well as the biometric recognition from different aspects can be monitored at the same time.

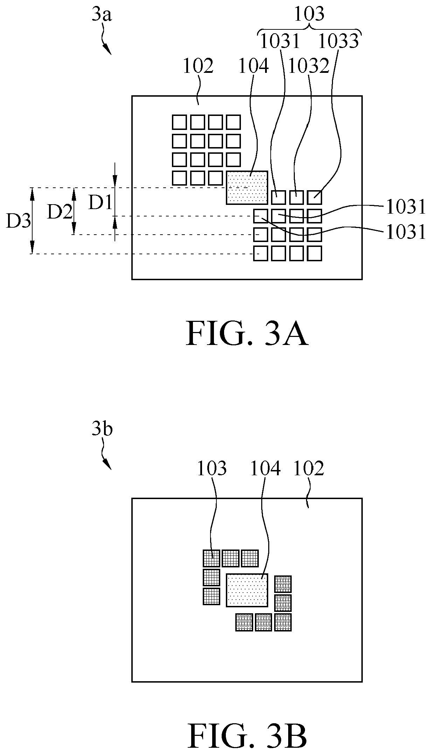

In one embodiment, the configuration of the light emitter 103 and the configuration of the light receivers 104 shown in FIG. 2A to 2F may be exchanged. For example, as shown in FIG. 3A, the arrangement is similar to that of the wearable optoelectronic sensing device 2f shown in FIG. 2F. In this embodiment, the wearable optoelectronic sensing device 3a includes a plurality of light emitters 103 arranged as two rectangular arrays, and the two rectangular arrays are arranged in a point symmetry with respect to the light receiver 104. Each of the rectangular arrays includes a plurality of first light emitters 1031, a plurality of second light emitters 1032, and a plurality of third light emitters 1033. The light receiver 104 of the wearable optoelectronic sensing device 3a is disposed on a geometric center of the two rectangular arrays, and the light receiver 104 is adapted to sense lights having at least three wavelengths. In one embodiment, the light receiver 104 is an array formed by a plurality of light receiving units. In one embodiment, a total area of the light receiver 104 is greater than an area of a single light emitter 103.

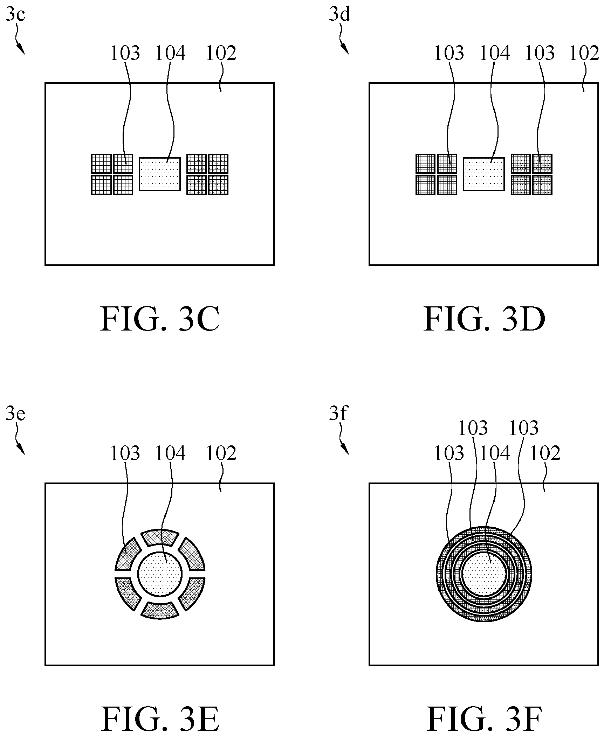

Please refer to FIG. 3B. In this embodiment, the wearable optoelectronic sensing device 3b includes a plurality of light emitters 103 arranged into two rectangular arrays. The two rectangular arrays are arranged in a point symmetry with respect to the light receiver 104. In this embodiment, the light emitter 103 of the wearable optoelectronic sensing device 3b may adopt an array-type light source to increase the density of the lights. Please refer to FIG. 3C. In this embodiment, the wearable optoelectronic sensing device 3c includes a plurality of light emitters 103 arranged into two rectangular arrays. The two rectangular arrays are arranged at two sides of the light receiver, and the two rectangular arrays are symmetrical with each other by taking an axis passing the geometric center of the light receiver 104 as the symmetry axis. Please refer to FIG. 3D. In this embodiment, the wearable optoelectronic sensing device 3d adopts the array-type light source to increase density of the light source. Please refer to FIG. 3E. In this embodiment, the plurality of light emitters 103 of the wearable optoelectronic sensing device 3e are arranged as a fan-shaped array, a plurality of fan-shaped arrays (FIG. 3E is six fan-shaped arrays) together forms a circular array, and the light receiver 104 is disposed on the center of circle of the circular array. The distance between the fan-shaped arrays can be adjusted according to different requirements. Please refer to FIG. 3F. In this embodiment, the plurality of light emitters 103 of the wearable optoelectronic sensing device 3f are arranged as an array formed in a concentric circle pattern, and the light receiver 104 is disposed on the center of circle of the concentric circle pattern.

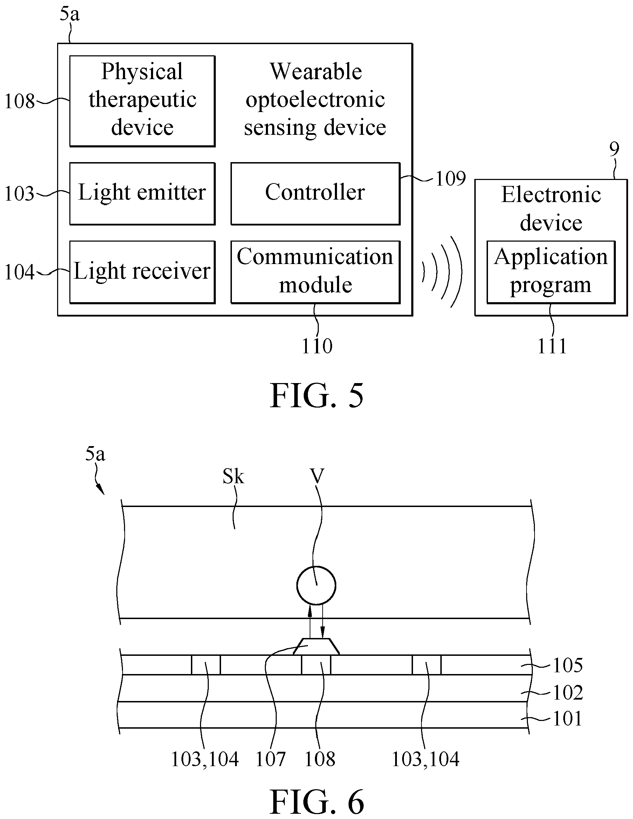

In one embodiment, the arrays formed by the plurality of light emitters 103 can be lighted on or off sequentially based on the order of rows or columns of the array or based on the distance between the arrays and the light receiver 104. For example, as shown in FIG. 3C, the array of the plurality of light emitters 103 at the left side, the right side, or both sides of the light receiver 104 is started to light on from the ten light emitters 103 at the leftmost column of the array. Then the ten light emitters 103 at the leftmost column of the array are lighted off, and ten light emitters 103 at a column next to the leftmost column of the array are lighted on. In subsequence, the light emitters 103 are lighted on in this sequence in the right direction. The light receiver 104 measures a plurality of physiological signals when the light emitters 103 in different columns are lighted on. Accordingly, in this embodiment, the controller 109 (shown in FIG. 5) coupled to the wearable optoelectronic sensing device 3c can determine the signal quality of the physiological signals from different light emitters 103 of respective different column so as to select physiological signals belonging to the specific column with a better quality (e.g., the physiological signals with highest signal-to-noise ratio). Alternatively, in one embodiment, the controller 109 (shown in FIG. 5) figures out the column where the light emitters 103 generate physiological signals with a better quality, and the controller 109 takes the light emitter 103 at that column and the light receiver 104 for subsequent physiological signal measurement. Accordingly, in this embodiment, when the user attaches the wearable optoelectronic sensing device 3c on the surface of the skin, the wearable optoelectronic sensing device 3c can identify the correct position of the vessels to obtain physiological signals with a best quality, thus the user does not need to aim the light emitters 103 or the light receiver 104 to the vessels precisely. Similarly, as shown in FIG. 3F, the array formed by the light emitters 103 can be lighted on or lighted off sequentially based on distance between the array and the light receivers 104 at the center of circle. Therefore, the light emitters 103 are lighted on from the innermost ring of the array or are lighted on from the outermost ring of the array. In the embodiment shown in FIG. 3A, the emission wavelengths of the arrays of the light emitters 103 at two diagonal sides of the light receiver 104 may be different from each other (for example, respectively 525 nm and 660 nm), so that the arrays with different emission wavelengths can be applied for measuring different physiological signals. Similarly, in the embodiment shown in FIG. 3C, the emission wavelengths of the arrays of the light emitters 103 at the right side and the left side of the light receiver 104 may be different from each other. Similarly, in the embodiment shown in FIG. 3E, the emission wavelengths of the arrays of the light emitters 103 at the upper side and the lower side of the light receiver 104 may be different from each other. In the embodiment shown in FIG. 3F, for each of the rings of the array of the light emitters 103, the emission wavelengths may be different from each other.

In one embodiment, the plurality of light emitters 103 adapted to emit lights having a shorter emission wavelength is disposed at positions nearer to the light receiver 104, and the plurality of light emitters 103 adapted to emit lights having a longer emission wavelength is disposed at positions farer from the light receiver 104. Specifically, as shown in FIG. 3A, in this embodiment, a first distance D1 is between the first light emitter 1031 and the light receiver 104, a second distance D2 is between the second light emitter 1032 and the light receiver 104, and a third distance D3 is between the third light emitter 1033 and the light receiver 104. The third distance D3 is greater than the second distance D2, and the second distance D2 is greater than the first distance D1. The emission wavelength of the third light emitter 1033 is greater than the emission wavelength of the second light emitter 1032, and the emission wavelength of the second light emitter 1032 is greater than the emission wavelength of the first light emitter 1031. In one embodiment, the emission wavelength may be defined as the wavelength of lights emitted by the light emitter 103 having a highest light intensity. In another embodiment, the emission wavelength may be defined as the wavelength range in which the light intensity of the light emitter is higher than a value, which is the peak power value of the light intensity distribution minus 3 dB. In one embodiment, the light receiver 104 is adapted to receive lights having at least three different wavelengths. For example, the dominant wavelength or the peak wavelength of the lights may be 525 nm, 660 nm, 940 nm, and/or 1300 nm. Each of the light emitters 1031, 1032, 1033 is adapted to emit lights having at least one emission wavelength. Moreover, the light emitters nearer to the light receiver 104 have a shorter emission wavelength, and the light emitters farer from the light receiver 104 have a longer emission wavelength. For example, the emission wavelength of the light emitters 1031 is 525 nm and/or 660 nm, the emission wavelength of the light emitters 1032 is 660 nm and/or 940 nm, and the emission wavelength of the light emitters 1033 is 1300 nm.

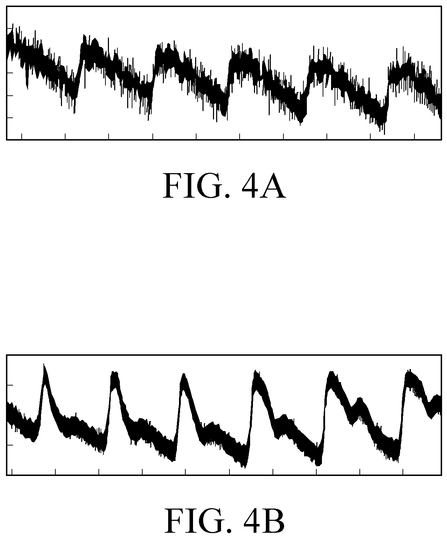

The transparent material layer 106 is not only applied to prevent the light emitter 103 and the light receiver 104 from contacting the skin of the organism, but also applied to increase the signal-to-noise ratio of the entire light sensing signals of the wearable optoelectronic sensing device. FIG. 4A is a PPG graph of a wearable optoelectronic sensing device 1a shown in FIG. 1 without the transparent material layer 106. In the case that the wearable optoelectronic sensing device 1a is devoid of the transparent material layer 106, the optoelectronic signals converted by the light receiver contain high-frequency noises. As a result, the PPG signals with such high-frequency noises is hard to be identified. FIG. 4B is a PPG graph of a wearable optoelectronic sensing device 1a shown in FIG. 1 with the transparent material layer 106. In the case that the wearable optoelectronic sensing device 1a includes the transparent material layer 106, the transparent material layer 106 can reflect ambient lights with larger incident angles. Hence, the ambient noise is reduced by the transparent material layer 106 and not received by the light receiver 104. Therefore, the PPG graph shown in FIG. 4B has a higher signal-to-noise ratio, and the physiological information such as the heartrate and the blood oxygen concentration can be identified easily. In another embodiment, the transparent material layer 106 and the cover layer 105 are made of the same material and are formed in a one-piece member. In other words, in this embodiment, the cover layer 105 of the wearable optoelectronic sensing device not only surrounds the side surfaces of the light emitter 103 and the side surfaces of the light receiver 104, but also covers the top surface of the light emitter 103 and the top surface of the light receiver 104.

The wearable optoelectronic sensing device 1a may be applied to wearable components for the organism, for example, may be applied to a knee cap, a wrist band, a kerchief, a scarf, a cuff, a glove, etc., and the wearable optoelectronic sensing device 1a may be attached to an article having a bent surface, such as the frame of the eyeglass. In one or some embodiments, the wearable optoelectronic sensing device 1a is flexible and has multiple measuring points. Therefore, the wearable optoelectronic sensing device 1a can be properly attached to the surface of the skin of the organism to increase the signal-to-noise ratio of the detection signals. Therefore, the commands of consumers who focus on the monitoring function of the products for physiological information can be satisfied.

FIG. 5 is a block diagram of a wearable optoelectronic sensing device according to an exemplary embodiment of the instant disclosure. In one embodiment, the system includes a wearable optoelectronic sensing device 5a and an electronic device 9. In this embodiment, the wearable optoelectronic sensing device 5a includes a light emitter 103, a light receiver 104, a physical therapeutic device 108, a controller 109, and a communication module 110. The physical therapeutic device 108 may be a phototherapeutic device or a thermotherapeutic device. The controller 109 may be a micro-control unit (MCU), an application specific integrated circuit (ASIC), a field programmable gate array (FPGA), or a logic circuit. The communication module 110 may be a wireless communication module adapted to transmit data to the electronic device 9. The communication module 110 may include, but not limited to, a signal transmission component which is applicable to global system for mobile communication (GSM), personal handy-phone system (PHS), code division multiple access (CDMA) system, wideband code division multiple access (WCDMA) system, long term evolution (LTE) system, worldwide interoperability for microwave access (WiMAX) system, wireless fidelity (Wi-Fi) system, or Bluetooth. The electronic device 9 may be a portable device or a cloud storage device. The electronic device 9 contains an application program 111 adapted to set parameters of the wearable optoelectronic sensing device 5a or to turn on or turn off the wearable optoelectronic sensing device 5a. In one embodiment, when the system shown in FIG. 5 is in operation, the electronic device 9, executing the application program 111, transmits an enabling signal to the communication module 110 of the wearable optoelectronic sensing device 5a. Then, the controller 109 of the wearable optoelectronic sensing device 5a enables the physical therapeutic device 108 to perform physical therapy, such as phototherapy, to the organism to be treated. During the physical therapy, the light emitter 103 and the light receiver 104 keep performing PPG signal measurements for the organism to monitor the changes of the physiological signal of the blood flow rate and/or the blood oxygen concentration. Then, the effect of the physical therapy can thus be checked by the PPG signal measurements. For example, the physical therapeutic device 108 emits lights to the vein of the organism, and the dominant wavelength or the peak wavelength of the lights is in a range between 600 nm and 640 nm. The PPG signals can be utilized to continuously determine whether the blood oxygen concentration is increased and whether the blood flow rate is increased. Hence, the PPG signals can be utilized to determine whether the time for the physical therapy is enough. The measurement signals of the wearable optoelectronic sensing device 5a during the physical therapy can be transmitted back to the electronic device 9 through the communication module 110 to determine whether the physical therapeutic device 108 should be kept turned on or turned off.

According to some embodiments, the physical therapeutic device 108 includes a therapeutic light emitter and a therapeutic light receiver to perform phototherapy to the subcutaneous tissues. The therapeutic light emitter may be a light emitting diode or a laser diode. The therapeutic light receiver may be, but not limited to, a photodiode, a photoresistor, or a visible or invisible light sensor. FIG. 6 is a schematic operational view of a wearable optoelectronic sensing device 5a according to an exemplary embodiment of the instant disclosure. As shown in FIG. 6, the wearable optoelectronic sensing device 5a includes a light emitter 103, a light receiver 104, and a physical therapeutic device 108. The physical therapeutic device 108 includes a therapeutic light emitter (not shown) and a therapeutic light receiver (not shown). The therapeutic light receiver is adapted to measure lights which are emitted to the vein by the therapeutic light emitter, and are absorbed and reflected by the vein. The controller (not shown) or the application program (not shown) checks whether the therapeutic lights of the therapeutic light emitter are correctly emitted to the vein V for therapy through the changes of the blood flow rate or the blood oxygen concentration in the vein V measured by the light emitter 103 and the light receiver 104. According to some embodiments, a transparent protruding spot 107 is disposed above the physical therapeutic device 108, and the position of the transparent protruding spot 107 corresponds to the position of the opening of the cover layer 105. The transparent protruding spot 107 may be adhered on the surface of the cover layer 105 or the surface of the physical therapeutic device 108 through optical clear adhesive or UV adhesive. The transparent protruding spot 107 may be a convex lens to allow the lights to be focused to a certain position of the skin Sk. In one embodiment, the configuration of the transparent protruding spot 107 allows the user of the wearable optoelectronic sensing device 5a to aim the transparent protruding spot 107 at the skin Sk through the user's touching, so that the light emitted from the therapeutic light emitter can be aimed at the capillary, the artery, the vein V, or the acupoint of the user through the transparent protruding spot 107. For example, the wearable optoelectronic sensing device 5a may be implemented as a wrist band, so that the wearable optoelectronic sensing device 5a can perform low energy vein laser therapy to the radial vein V and the ulnar vein V. Therefore, effects of blood activation, increasing of the oxygenation of blood cells, and neuropathy repairment can be achieved.

According to some embodiments, the emission wavelength of the therapeutic light emitter and the emission wavelength of the light emitter 103 are different from each other to prevent interferences between the lights. According to some embodiments, the controller 109 controls the therapeutic light emitter and the light emitter 103 to emit lights alternately to prevent interferences between the lights. According to some embodiments, the emission wavelength of the therapeutic light emitter is in a range between 600 nm and 640 nm. According to some embodiments, the emission wavelength of the therapeutic light emitter is 632.8 nm to provide a better phototherapy effect. The wearable optoelectronic sensing device 5a may determine whether the therapy is finished during the phototherapy procedure through the light sensing signals detected by the light receiver 104. For example, during the phototherapy, the application of the intravascular laser increases the oxygen content of the erythrocytes, and this physiological feature is presented by the change of the absorption of the light sensing signals at different wavelength ranges. Therefore, when the wearable optoelectronic sensing device 2a determines that the intensity of the light sensing signals at a certain wavelength range reaches the lower limit or the upper limit of a preset intensity range (e.g., the absorption of the light at 940 nm which can present the oxidative hemoglobin increases) to check that the therapy is finished. According to some embodiments, when the wearable optoelectronic sensing device 5a determines that the intensity of the light sensing signals at a certain wavelength range reaches the lower limit or the upper limit of the preset intensity range, the wearable optoelectronic sensing device 5a can enable the therapeutic light emitter correspondingly.

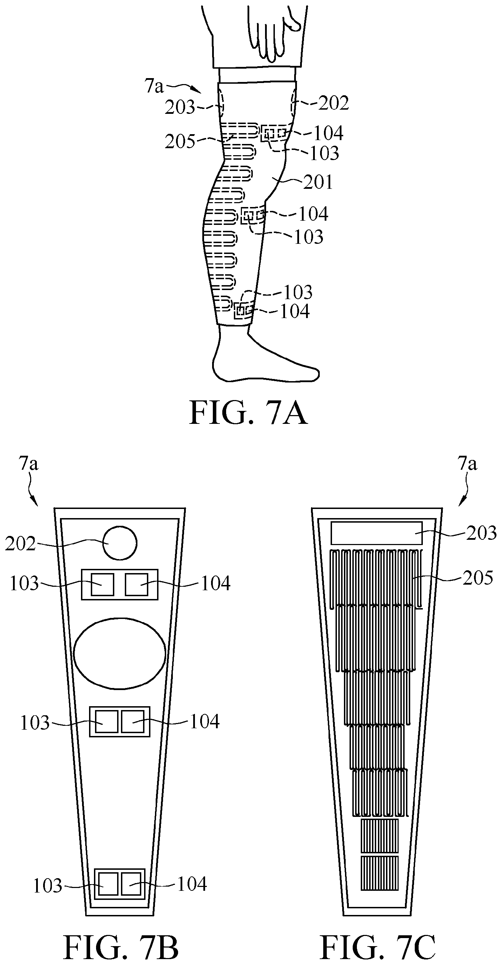

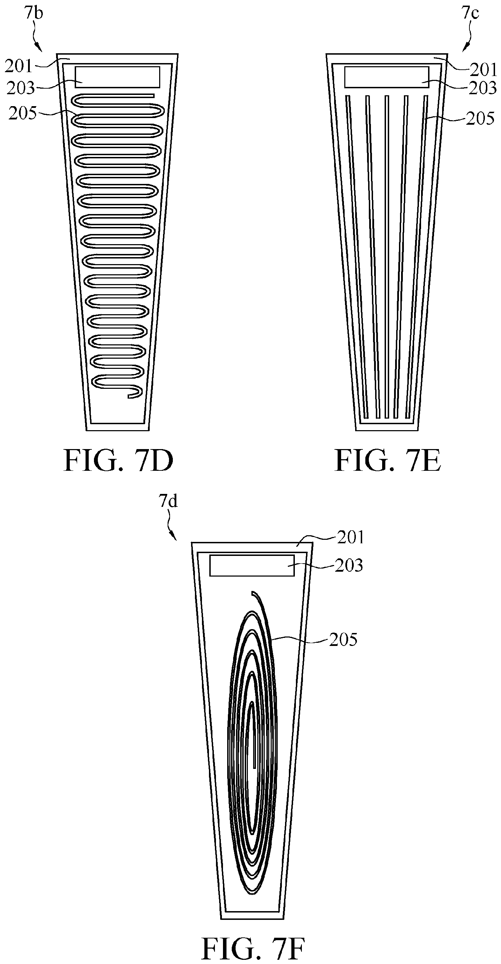

In another embodiment, the physical therapeutic device of the wearable optoelectronic sensing device may be a thermotherapeutic module and is applicable for sporting wears. During exercise, the discomfort caused by accumulations of lactic acid in the muscle or by cold extremities can be mitigated through the thermotherapy to reduce risks of sports injury. FIG. 7A to FIG. 7C illustrate schematic views of a knee cap having a wearable optoelectronic sensing device according to an exemplary embodiment of the instant disclosure. As shown in FIG. 7A, the wearable optoelectronic sensing device 7a may be implemented as a knee cap 201. The wearable optoelectronic sensing device 7a can be detached from the knee cap 201, so that the knee cap 201 can be washed conveniently. In this embodiment, the wearable optoelectronic sensing device 30 may include a switch 202, a battery 203, a light emitter 103, a light receiver 104, and a thermotherapeutic module 205. The thermotherapeutic module 205 may be a heater including a heatable metal wire. The switch 202 is adapted to turn on or turn off the wearable optoelectronic sensing device 7a. In one embodiment, the switch 202 is disposed on a front side of the knee cap 201, so that the user can press the switch 202 conveniently. Moreover, in this embodiment, with such configuration, when the user bends his/her lower limbs, the user can be prevented from contacting the switch 202 unintentionally. The battery 203 is disposed on the rear side of the knee cap 201, and the battery 203 may be a mercury battery, a lithium battery, a solar cell, a soft battery, a thermal cell, or a piezoelectric cell. Please refer to FIG. 7B, which illustrate a schematic view showing the front side of the knee cap 201. It is understood that, there are many microvascular vessels distributed in the superficial skin of the front side of the lower limbs of the human body. Hence, the light emitter 103 and the light receiver 104 can be disposed on the front side of the knee cap 201 to perform measurements of physiological signals for phtoplethysmography (PPG) or to perform biometric recognition. FIG. 7C is a schematic view showing the rear side of the knee cap 201. It is understood that, there are many muscular tissues distributed in the rear side and lateral sides of the lower limbs of the human body. Hence, the thermotherapeutic module 205 may be disposed on the rear side of the knee cap 201 to cover the rear side and lateral sides of the lower limbs, so that the metabolism of the lactic acid can be accelerated or the muscles of the human body can be warmed up through the heating of the thermotherapeutic module 205. In one embodiment, the thermotherapeutic module 205 may be disposed on a portion of the knee cap 201 near the skin, so that the thermotherapeutic module 205 can perform therapy to the muscles (such as gluteus maximus, semitendinosus, gastrocnemius, etc.). In one embodiment, the thermotherapeutic module 205 may be a metal plate or metal wire with high impedance. As shown in FIG. 7C, the metal wire is a structure extending along the longitudinal axis of the knee cap 201 and wound on the rear side of the knee cap 201 in the form of bent "N" profile, such that the contact area between the metal wire and the skin can be increased. FIG. 7D to FIG. 7F illustrate schematic rear views of knee caps having wearable optoelectronic sensing devices according to different embodiments of the instant disclosure. FIG. 7D is the thermotherapeutic module 205 on the rear side of the wearable optoelectronic sensing device 7b implemented as the knee cap 201. In this embodiment, the metal wire of the thermotherapeutic module 205 is wound on the rear side of the knee cap 201 in the form of bent "S" profile. FIG. 7E is the thermotherapeutic module 205 on the rear side of the wearable optoelectronic sensing device 7c implemented as the knee cap 201. In this embodiment, the metal wires of the thermotherapeutic module 205 are arranged as elongated bars covering on the rear side of the knee cap 201. FIG. 7F is the thermotherapeutic module 205 on the rear side of the wearable optoelectronic sensing device 7d implemented as the knee cap 201. In this embodiment, the metal wire of the thermotherapeutic module 205 is arranged as a spiral structure covering on the rear side of the knee cap 201. Therefore, according to these embodiments, the metal wires can completely cover the rear side and lateral sides of the lower limb.

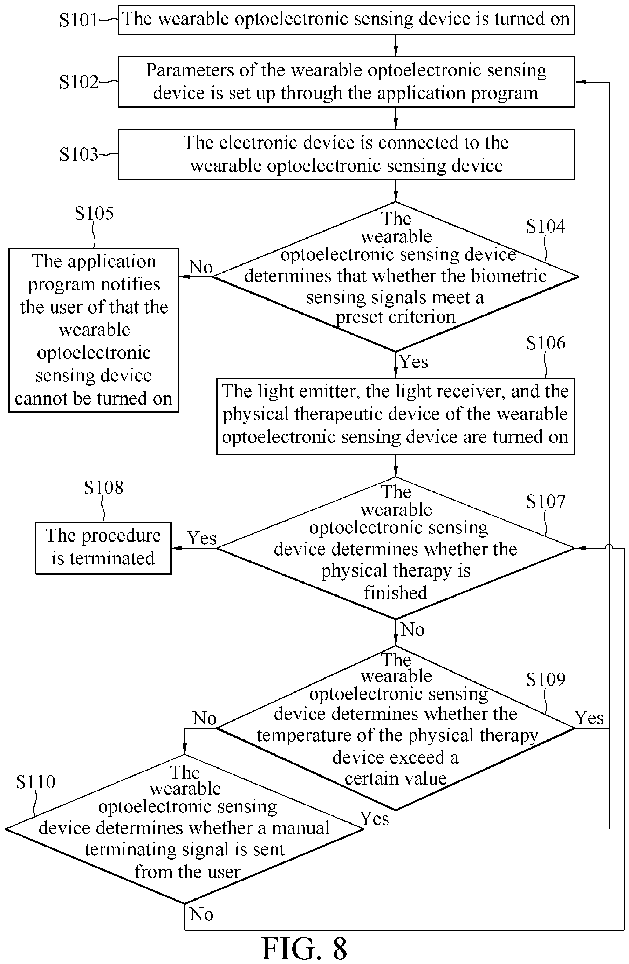

FIG. 8 is an operation flowchart of a wearable optoelectronic sensing device according to an exemplary embodiment of the instant disclosure. In this embodiment, the user turns on the wearable optoelectronic sensing device through the switch (the step S101). Then, the user inputs parameters to set the wearable optoelectronic sensing device through the application program of the electronic device (e.g., the mobile phone) (the step S102). The electronic device, e.g., a mobile phone, is connected to the wearable optoelectronic sensing device through the communication module (the step S103). Then, the wearable optoelectronic sensing device determines that whether the biometric sensing signals meet a preset criterion (the step S104). In this embodiment, the preset criterion may indicate that the wearable optoelectronic sensing device is properly attached to the to-be-detected portion of the organism, and/ or indicate that the to-be-detected organism is performing a preset activity. In one embodiment, the detection for these two criteria may be checked by the intensity of the PPG signal. When the wearable optoelectronic sensing device determines that the sensing signal does not meet the preset criterion (the determination result is "no"), through allowing the electronic device to display an image, to perform vibration, to generate sounds, the application program notifies the user of that the wearable optoelectronic sensing device cannot be turned on (the step S105). When the wearable optoelectronic sensing device determines that the sensing signal meets the preset criterion (the determination result is "yes"), the light emitter, the light receiver, and the physical therapeutic device of the wearable optoelectronic sensing device are turned on (the step S106). In this embodiment, the physical therapeutic device is adapted to perform physical therapy such as thermotherapy, phototherapy, and sound therapy.

The wearable optoelectronic sensing device can determine whether the physical therapy is finished through the PPG physiological signals (the step S107). For example, in the thermotherapy, temperature increasing leads blood vessel expansion so as to accelerate the heartrate, and such feature can be presented in the PPG signals. Therefore, when the wearable optoelectronic sensing device determines that the light sensing signal reaches the upper limit of the preset value, the wearable optoelectronic sensing device determines that the therapy is finished. On the other hand, when the wearable optoelectronic sensing device determines that the light sensing signal reaches the lower limit of the preset value, the wearable optoelectronic sensing device may continue turning on the physical therapeutic device. When the wearable optoelectronic sensing device determines that the therapy is finished (the determination result is "yes") (the step S107), the procedure is terminated (the step S108), and the application program can notify the user of the termination of the procedure. When the wearable optoelectronic sensing device determines that the therapy is not finished (the determination result is "no"), the physical therapy is kept performing. Alternatively, in one embodiment, the wearable optoelectronic sensing device may include a temperature sensor. During the thermotherapy, the temperature sensor is adapted to detect the temperature of the physical therapeutic device to determine that whether the physical therapeutic device is overloaded. When the temperature is determined to exceed a certain value (the determination result is "yes") (the step S109), the physical therapy is terminated and the temperature parameter is allowed to be set again through the application program. When the temperature is determined not to exceed the certain value (the determination result is "no") (the step S109), the physical therapy is kept performing. During the therapy, when the wearable optoelectronic sensing device receives a manual terminating signal from the user (the determination result is "yes") (the step S110), for example, when the user presses the switch on the wearable optoelectronic sensing device or taps the pause button of the application program, the physical therapy is terminated and parameters of the application program 111 are allowed to be set again. On the other hand, when the wearable optoelectronic sensing device does not receive the manual terminating signal (the determination result is "no") (the step S110), the physical therapy is kept forming. It should be noted that, the aforementioned steps do not need to be executed in order. For example, the step S102 and the step S103 may be exchanged, or for example, the step S109 and the step S110 may be exchanged. For example, when the determination result in the step S109 or the step S110 is "yes", the step S108 is executed.

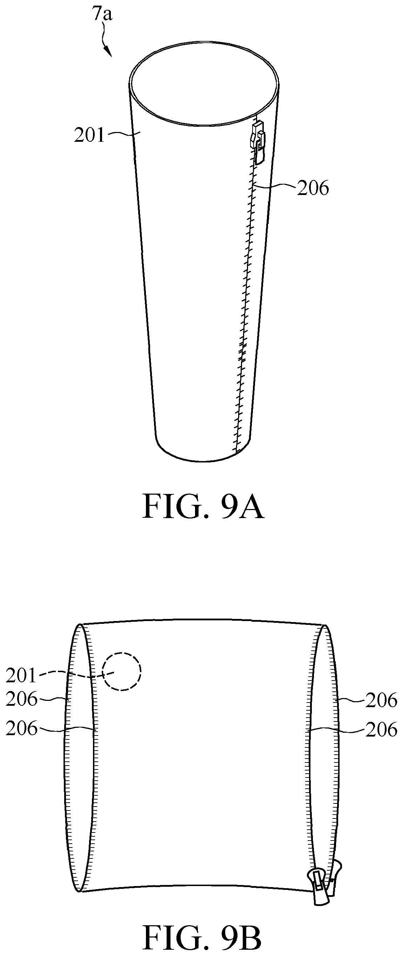

FIG. 9A and FIG. 9B illustrate schematic views of a connection member of the knee cap according to an exemplary embodiment of the instant disclosure. Please refer to FIG. 9A, in this embodiment, the knee cap 201 includes a connection member 206, so that the user can put on and take off the knee cap 201 conveniently. The connection member 206 may be a zipper, a magnet, a button, or a snap fastener. The number of the connection member, such as the zipper or the buttons, may be one or formed a set of connection members. In another embodiment, as shown in FIG. 9B, the knee cap 201 may be formed in a closed loop structure. The closed loop structure includes a receiving space for the lower limb and two connecting openings which are opposite to each other. Each connecting opening includes two sets of connection members 206. Therefore, in this embodiment, when the user puts on or takes off the knee cap 201, the connection members 206 at the openings can be attached to other fabrics of the knee cap 201.

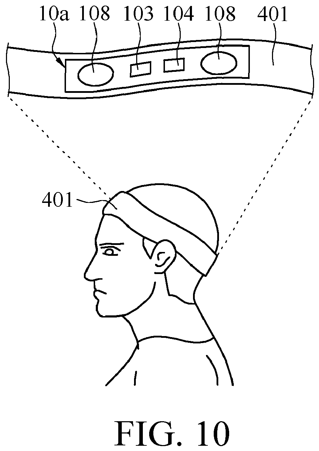

FIG. 10 is a schematic operational view of a kerchief having a wearable optoelectronic sensing device according to an exemplary embodiment of the instant disclosure. The wearable optoelectronic sensing device 10a may be implemented as a kerchief 401. In this embodiment, the wearable optoelectronic sensing device 10a includes a light emitter 103, a light receiver 104, and a physical therapeutic device 108. The kerchief 401 is adapted to cover the forehead of the user, or may be adapted to cover the whole head, the neck, or the four limbs of the user. The physical therapeutic device 108 may be a thermotherapeutic module comprising a heatable metal wire. Upon the user uses the kerchief 401 to perform thermotherapy, the temperature increasing makes the user relax, and such physiological feature can be checked through the measurements of the PPG signals performed by the light emitter 103 and the light receiver 104, so that the therapy effect can be checked. Hence, through the result of the therapy effect, the user can determine whether the physical therapeutic device 108 is allowed to continue performing the therapy. In another embodiment, the wearable optoelectronic sensing device 10a further includes a brainwave sensor (not shown), and the brainwave sensor is adapted to be utilized along with the PPG signals for determining the therapy effect.

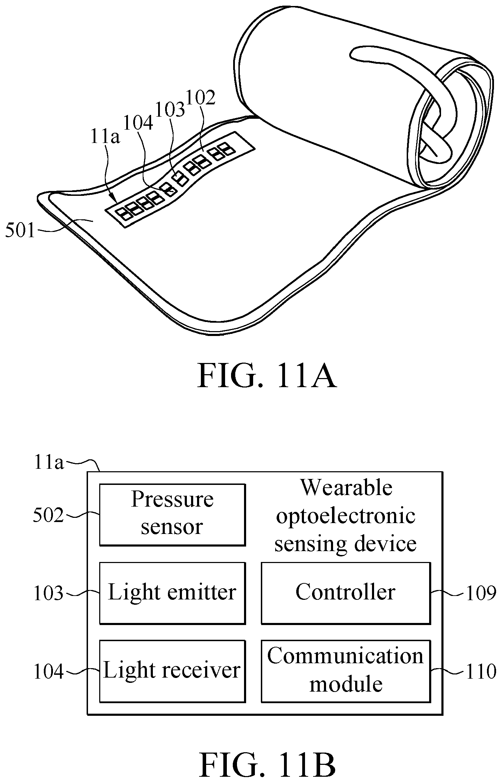

FIG. 11A is a schematic view of a cuff having a wearable optoelectronic sensing device according to an exemplary embodiment of the instant disclosure. FIG. 11B is a block diagram of a wearable optoelectronic sensing device according to another exemplary embodiment of the instant disclosure. As shown in FIG. 11A, the wearable optoelectronic sensing device 11a may be implemented as a cuff 501. The cuff 501 may include an airbag (not shown) and a motor (not shown). The wearable optoelectronic sensing device 11a may include a substrate layer 102, a plurality of light emitters 103, a plurality of light receivers 104, a pressure sensor 502 (as shown in FIG. 11B), a controller 109 (as shown in FIG. 11B), and a communication module 110 (as shown in FIG. 11B). The airbag (not shown) may be disposed in the cuff 501 for pressing the upper limb to perform blood pressure measurement. The motor (not shown) may be connected to the airbag through inflating and discharging piping to achieve the pressure increasing or decreasing of the airbag. The light emitters 103 and the light receivers 104 may be disposed on the surface of the substrate layer 102. The pressure sensor 502 may be disposed on the surface of the substrate layer 102. In another embodiment, the pressure sensor 502 is disposed on the textile layer (not shown) under the substrate layer 102. In another embodiment, the pressure sensor 502 may be disposed on the inner side of the airbag of the cuff 501 or may be disposed on the inner side of the inflating and discharging piping connected to the airbag. The pressure sensor 502 may be, but not limited to, an acoustic wave sensor, a barometer, a vibration sensor, a capacitive pressure sensor, or a resistive pressure sensor. For example, the pressure sensor 502 is a vibration sensor disposed on the substrate layer 102. In this embodiment, when the cuff 501 loosens from the tightened state, the vibration of the cuff 501 caused by the changes of the pulse of the object can be detected by the pressure sensor 502. For example, the pressure sensor 502 measures the air pressure of the charging and discharging pipelines. When the cuff 501 loosens from the tightened state, the change of the air pressure inside the airbag of the cuff 501 caused by the changes of the pulse of the object is detected by the pressure sensor 502. According to some embodiments, the light emitters 103 and the light receivers 104 are disposed on the inner side of the cuff 501. Therefore, when the cuff 501 surrounds the upper limb of the user to perform the blood pressure measurement, the light emitters 103 and the light receivers 104 can be attached on the skin of the to-be-detected organism. Alternatively, in one embodiment, the wearable optoelectronic sensing device 11a may further include an electrocardiography (ECG) sensor to monitor the electrocardiography signals. Moreover, through measuring the electrocardiography signals and the blood pressure, the wearable optoelectronic sensing device 11a can perform a long-term monitoring of physiological signals for the user.

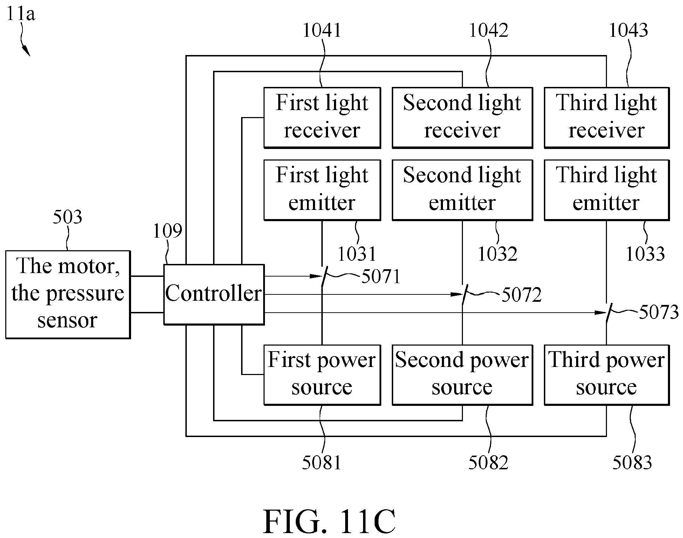

FIG. 11C is a schematic circuit block diagram of a cuff having a wearable optoelectronic sensing device according to an exemplary embodiment of the instant disclosure. In this embodiment, the controller 109 is coupled to the motor, the pressure sensor 503, a plurality of light receivers 1041, 1042, 1043, a plurality of switches 5071, 5072, 5073, and a plurality of power sources 5081, 5082, 5083. The first switch 5071 is connected to the first power source 5081 to supply electricity for the first light emitter 1031. The second switch 5072 is connected to the second power source 5082 to supply electricity for the second light emitter 1032. The third switch 5073 is connected to the third power source 5083 to supply electricity for the third light emitter 1033. Therefore, the controller 109 can control the motor, the pressure sensor 503, and the light emitters 1031, 1032, 1033 independently or at the same time. Then, the controller can sequentially turn on or turn off the light emitters 1031, 1032, 1033. According to some embodiments, the user can wear the cuff 501 for a long time, the light emitters 1031, 1032, 1033 and the light receivers 1041, 1042, 1043 of the wearable optoelectronic sensing device 11a can be utilized to monitor the blood pressure for a long time. Especially, in such embodiment, the user may wear the cuff 501 at nighttime to measure the light sensing signals to obtain the blood pressure signals without affecting the sleeping by the noises caused by the airbag of the cuff 501. In one embodiment, the light sensing signals measured by the light receivers 1041, 1042, 1043 can be converted into blood pressure signals through a conversion equation. The conversion equation may be a regression function or may be a neuro network model. When the conversion equation is needed to be calibrated, the controller 109 may control the motor and the pressure sensor 203 of the cuff 501 to perform the blood pressure measurement, so that the conversion equation can be calibrated according to the blood pressure data measured by the cuff 501. For example, the wearable optoelectronic sensing device 11a controls the motor and the pressure sensor 503 to perform the blood pressure measurement to obtain a label data. The wearable optoelectronic sensing device 11a also utilizes the light emitters 1031, 1032, 1033 and the light receivers 1041, 1042, 1043 to obtain a plurality of light sensing signals as the training data, and the network state of the neuro network model can be updated based on the label data and the training data.

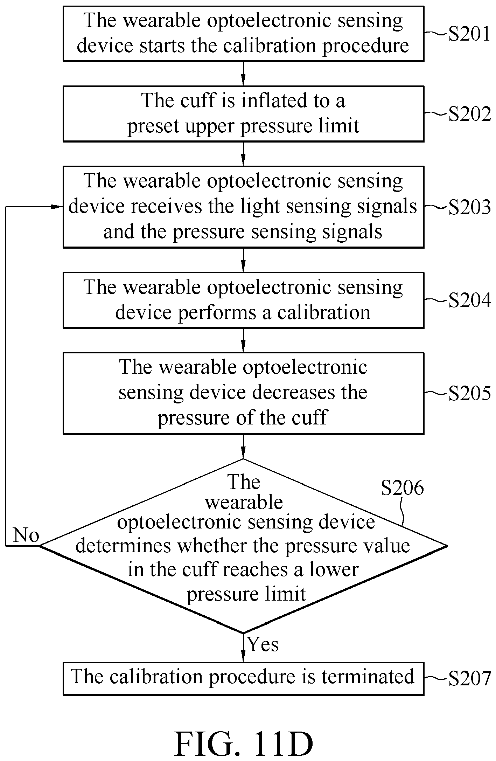

FIG. 11D is a flowchart of a calibration procedure of the cuff having the wearable optoelectronic sensing device according to an exemplary embodiment of the instant disclosure. In this embodiment, after the wearable optoelectronic sensing device starts the calibration procedure (the step S201), the cuff is inflated to a preset upper pressure limit (the step S202). In this embodiment, the upper pressure limit is the maximum pressure value among the to-be-tested pressure values. Then, the wearable optoelectronic sensing device can receive the light sensing signals from the light receiver and the pressure sensing signals from the pressure sensor at the same time (the step S203), and then the wearable optoelectronic sensing device performs a calibration based on the light sensing signals and the pressure sensing signals (the step S204). For example, the wearable optoelectronic sensing device calibrates the parameters of the regression function or adjust the weighting or deviation of the neuro network model based on the measured values. After the maximum pressure value is calibrated, the wearable optoelectronic sensing device decreases the pressure of the cuff to a preset value (the step S205) and determines whether the pressure value in the cuff reaches a lower pressure limit (the step S206). When the wearable optoelectronic sensing device determines that the pressure of the cuff reaches the lower limit (the determination result is "yes") (the step S206), the calibration procedure is terminated (the step S207). When the wearable optoelectronic sensing device determines that the pressure of the cuff does not reach the lower limit (the determination result is "no") (the step S206), the procedures in the steps S203 to S205 are repeated.

For example, for the cuff, the preset upper pressure limit is set to be 250 mm Hg, the preset pressure decrease interval is 10 mm Hg, and the preset lower pressure limit is set to be 20 mm Hg. When the step S202 is executed, the wearable optoelectronic sensing device adjusts the pressure of the cuff to be 250 mm Hg and then executes the step S203 and the step S204. Next, the pressure of the cuff is decreased by 10 mm Hg (the step S205). Hence, the pressure in the cuff is 240 mm Hg, and the wearable optoelectronic sensing device determines that the pressure of the cuff does not reach the preset lower pressure limit (which is 20 mm Hg) (the step S206), and thus executes the steps S203 to S205 again. It should be noted that, the aforementioned steps do not need to be executed in order. For example, the step S204 and the step S205 may be exchanged. For example, in the step S204 of calibrating the conversion equation, when it is determined that the measured values are incorrect, the step S203 is repeatedly executed. Moreover, the pressure adjustment may be changed from the incremental pressure decrease to the incremental pressure increase. For example, the step S202 may be changed as inflating the pressure of the cuff to be a lower pressure limit, the step S205 may be changed as increasing the pressure of the cuff, and the step S206 may be changed as determining that whether the pressure of the cuff reaches the upper pressure limit.

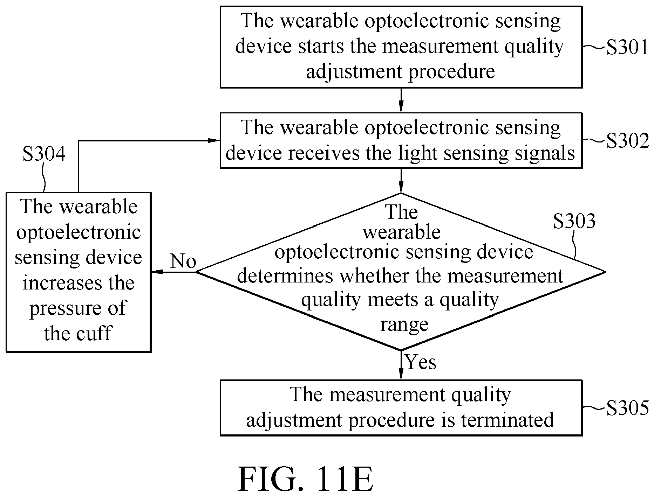

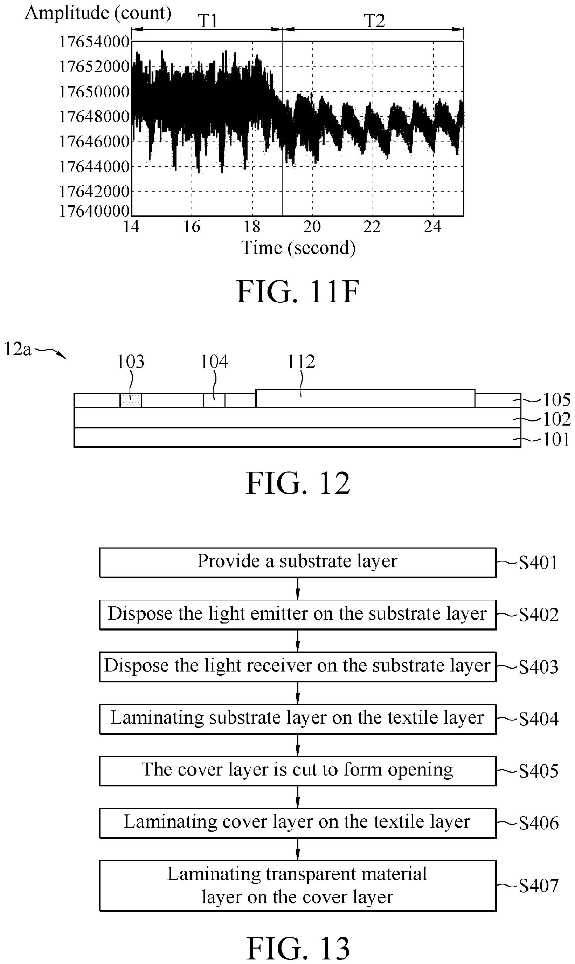

According to some embodiment, when the signal quality of the light sensing signals is poor, the wearable optoelectronic sensing device applied to the cuff may execute a measurement quality adjustment procedure. FIG. 11E is a flowchart of a measurement quality adjustment procedure of the cuff having the wearable optoelectronic sensing device according to an exemplary embodiment of the instant disclosure. According to some embodiments, the wearable optoelectronic sensing device starts the measurement quality adjustment procedure (the step S301) and receives the light sensing signals (the step S302). The wearable optoelectronic sensing device determines that the measurement quality meets a quality range, for example, determines that whether the signal-to-noise ratio of the light sensing signals in the target wavelength range is higher than a preset value. The noise may be stray light from the environments or due to that the gap between the cuff and the skin is too large. When the wearable optoelectronic sensing device determines that the measurement quality meets the quality range (the determination result is "yes") (the step S303), the measurement quality adjustment procedure is terminated (the step S305). When the wearable optoelectronic sensing device determines that the measurement quality does not meet the quality range (the determination result is "no") (the step S304), the pressure of the cuff is increased by a preset value (the step S304). Therefore, the light emitter and the light receiver at the inner side of the cuff can further attach to the surface of the skin of the user, so that the gap between the cuff and the skin can be reduced, and the lights emitted by the light emitter can be transmitted to the skin of the user more easily. Then, the wearable optoelectronic sensing device executes the step S302 again. FIG. 11F is a graph showing signals before and after the measurement quality adjustment procedure according to an exemplary embodiment of the instant disclosure. As shown in FIG. 11F, in the duration T1 which is before the measurement quality adjustment procedure, the light sensing signals contain a lot of noises to shield the target signals. In the duration T2 which is after the procedure, the noises are greatly reduced.

FIG. 12 is a schematic view of a wearable optoelectronic sensing device having a display panel according to an exemplary embodiment of the instant disclosure. The wearable optoelectronic sensing device 12a includes a substrate layer 102. A display panel 112 covers the surface of the substrate layer 102 and is flexible, such as a micro LED panel or an OLED panel. The display panel may show the physiological information or parameter setting information of the wearable optoelectronic sensing device 12a for the user, thus the convenience for using the device is enhanced. According to some embodiments, the wearable optoelectronic sensing device 12a may be implemented as a glove (not shown). The glove (not shown) may include a display panel 112, a substrate layer 102, a light emitter 103, and a light receiver 104. The light emitter 103 and the light receiver 104 may be disposed on the finger pulp of the glove or on the wrist of the glove for detecting the capillary at the end portion of the finger or the vein at the wrist. The display panel 112 may be disposed on the back of the glove, so that the user can operate the display panel 112 conveniently. Alternatively, in one embodiment, the glove includes a thermotherapeutic module (not shown) at the palm of the glove to perform thermotherapy to the muscles of the palm (e.g., the thenar muscle). Alternatively, in one embodiment, the glove includes a phototherapeutic module (not shown) at the palm or the wrist of the glove to perform phototherapy to the acupoint on the palm or the vein of the wrist. Alternatively, in one embodiment, the glove includes a piezoelectric sensor (not shown), the piezoelectric sensor may be disposed on a contact surface between the glove and the skin, the wrist, or the finger portion of the glove, so that the piezoelectric sensor can detect the motion or the vibration of the hand. The light sensing signals is compared with the piezoelectric sensing signals or performed differential processing with the piezoelectric sensing signals. Alternatively, in one embodiment, the glove includes a solar cell (not shown). The solar cell may be disposed on the back of the glove. Therefore, during daily use, the ambient lights are not shielded by the hand easily and used to charge electricity to the wearable optoelectronic sensing device. Alternatively, in one embodiment, the glove includes a biometric recognizer at the fingertip of the glove, for example, the biometric recognition may be carried out by recognizing the distribution of the capillaries at the fingertip with the light emitter 103 and the light receiver 104. Alternatively, the glove includes a UV light sterilization module. Therefore, when the glove contacts an object, the glove can perform UV light sterilization to the surface of the object through the lightguide structure on the glove.

FIG. 13 is a manufacturing flowchart of a wearable optoelectronic sensing device according to an exemplary embodiment of the instant disclosure. In this embodiment, first, a substrate layer is provided (the step S401), and the light emitter and the light receiver are disposed on the substrate layer (the step S402 and the step S403). Next, the substrate layer is laminated on the textile layer (the step S404). Then, the cover layer is cut to form openings (the step S405), where the positions of the openings on the cover layer correspond to the positions of the light emitter and the light receiver on the substrate layer, and the cover layer is laminated on the textile layer (the step S406). Therefore, the substrate layer 102 is sandwiched between the cover layer 105 and the textile layer 101. According to some embodiments, the transparent material layer is laminated on the cover layer (the step pS407). The lamination may be achieved by thermal lamination or may be achieved by applying adhesive between the layers followed by the lamination procedure. In another embodiment, the light emitter and the light receiver may be adhered to the transparent material layer through optical clear adhesive to prevent the air gap therebetween to affect light transmission. It is understood that, the aforementioned steps do not need to be executed in order. For example, the step S405 may be executed in advance, as long as it is ensured that the positions of the openings correspond to the positions of the light emitter and the light receiver on the substrate layer. According to some embodiment, the manufacturing method for the wearable optoelectronic sensing device may adopt the printing process. In another embodiment, the cover layer may be directly formed above the substrate layer to cover the side surfaces and the top surface of the light emitter and the side surfaces and the top surfaces of the light receiver, and thus in this embodiment the transparent material layer is not needed. The formation method of the cover layer may be applying a film, coating, glue dipping, or casting.

As above, according to some embodiments, the wearable optoelectronic sensing device is flexible and can measure multiple points on the organism, so that the wearable optoelectronic sensing device can be applied to different daily supplies. Moreover, the array configuration and the signal calibration of the light emitter and the light receiver can enhance the signal-to-noise ratio of the measurement signals effectively, thus satisfying the consumer requirements in healthy monitor markets. According to some embodiments, the wearable optoelectronic sensing device provides both physiological monitoring and thermotherapy function, so that the wearable optoelectronic sensing device can be applied to monitor the blood pressure and the blood oxygen concentration of the user during exercise and to warm up the limbs of the user to accelerate the metabolism of the lactic acid and reduce the sports injury. According to some embodiments, the wearable optoelectronic sensing device provides both physiological monitoring and phototherapy function, so that the wearable optoelectronic sensing device can be applied to portable and household therapies, and thus the wearable optoelectronic sensing device is adapted to activate the bloods and to repair the neuropathy disorder. Moreover, the wearable optoelectronic sensing device can perform the therapy and monitor the effect of the therapy at the same time. According to some embodiments, the wearable optoelectronic sensing device provides a brainwave monitoring function, and the wearable optoelectronic sensing device is adapted to monitor a sleeping state or a breathing function, According to some embodiments, the wearable optoelectronic sensing device provides a blood pressure monitoring function, and the wearable optoelectronic sensing device can be utilized along with a cuff to perform calibration to the wearable optoelectronic sensing device. Hence, the measurement accuracy can be increased and the interference to the user can be reduced. Moreover, in some embodiments, when the signal quality of the light sensing signals is poor, the wearable optoelectronic sensing device allows the pressure adjustment for the cuff, so that the light receiver can be attached closer to the surface of the skin, thus improving the measurement quality.

## Claims

1. A wearable optoelectronic sensing device, comprising:
a textile layer;
a substrate layer disposed on the textile layer;
a light emitter disposed on a surface of the substrate layer;
a plurality of light receivers disposed on the surface of the substrate layer and formed in an array, the light emitter disposed at a geometric center of the array, the plurality of light receivers comprising:
a first light receiver adapted to sense a first sensing wavelength;
a first distance between the first light receiver and the light emitter;
a second light receiver adapted to sense a second sensing wavelength greater than the first sensing wavelength; and
a second distance between the second light receiver and the light emitter, and greater than the first distance; and
a cover layer disposed on the substrate layer, and comprising a plurality of openings;
wherein each of the plurality of openings has a position corresponding to the light emitter and the light receiver.

2. The wearable optoelectronic sensing device according to claim 1, further comprising a transparent material layer disposed on the cover layer.

3. The wearable optoelectronic sensing device according to claim 2, wherein a refractive index of the transparent material layer is in a range between 1.42 and 1.55.

4. The wearable optoelectronic sensing device according to any one of the preceding claims, further comprising a plurality of transparent protruding dots disposed on the cover layer, wherein each of the plurality of transparent protruding dots has a position corresponding to at least one of the openings.

5. The wearable optoelectronic sensing device according to any one of the preceding claims, further comprising a physical therapeutic device, and a communication module, wherein the light emitter is coupled to the communication module.

6. The wearable optoelectronic sensing device according to claim 5, wherein the physical therapeutic device comprises a therapeutic light emitter and a therapeutic light receiver; the light emitter is adapted to emit a first light, the therapeutic light emitter is adapted to emit a second light which has an emission wavelength different from that of the first light.

7. The wearable optoelectronic sensing device according to claim 6, wherein the emission wavelength of the second light is in a range between 600 nm and 640 nm.

8. The wearable optoelectronic sensing device according to claim 5, wherein the physical therapeutic device comprises a plurality of therapeutic light emitters disposed on the surface of the substrate layer, wherein the plurality of therapeutic light emitters is configured to emit light to an acupoint of the user.

9. The wearable optoelectronic sensing device according to claim 5, wherein the wearable optoelectronic sensing device is disposed on a knee cap, the physical therapeutic device is a heater, the light emitter and the plurality of light receivers are disposed on a front side of the knee cap, and the heater is disposed on a rear side of the knee cap.

10. The wearable optoelectronic sensing device according to claim 9, wherein the heater is of an elongated, spiral, or curved structure extending along a longitudinal axis of the knee cap.

11. The wearable optoelectronic sensing device according to claim 9 or 10, wherein the textile layer is formed as a closed loop structure which comprises a receiving space and two connecting openings, and the two connecting openings are opposite to each other; each connecting opening comprises two sets of connection members, and the substrate layer is disposed in the receiving space.

12. The wearable optoelectronic sensing device according to any one of the preceding claims, wherein the wearable optoelectronic sensing device is disposed on a cuff; the wearable optoelectronic sensing device further comprises a controller and a pressure sensor; the controller is adapted to:
receive a light sensing signal from at least one of the plurality of light receivers;
convert the light sensing signal into a blood pressure signal according to a conversion equation;
receive a pressure sensing signal from the pressure sensor; and
calibrate the conversion equation according to the pressure sensing signal.

13. The wearable optoelectronic sensing device according to claim 12, wherein the controller is adapted to:
adjust a plurality of pressure states of the cuff;
receive the light sensing signal from at least one of the light receivers and receive the pressure sensing signal from the pressure sensor in each of the pressure states; and
calibrate the conversion equation according to the pressure sensing signal.

14. The wearable optoelectronic sensing device according to any one of the preceding claims, further comprising a flexible panel disposed on the surface of the substrate layer.

15. The wearable optoelectronic sensing device according to any one of the preceding claims, wherein the first sensing wavelength is 525 nm, the second sensing wavelength is 660 nm; the plurality of light receivers comprises a third light receiver adapted to sense a third sensing wavelength which is 940 nm; a third distance greater than the second distance is between the third light receiver and the light emitter.
